# NEUE EUROPÄISCHE PATENTSCHRIFT

(11) **EP 1 102 550 B2**
(45) Veröffentlichungstag und Bekanntmachung des Hinweises auf die Entscheidung über den Einspruch: **21.11.2018**
(45) Hinweis auf die Patenterteilung: 12.10.2005
(21) Anmeldenummer: 99952243.6
(22) Anmeldetag: 04.08.1999
(51) Int. Cl.: A23L 1/36, A23L 1/38

(54) **VERFAHREN ZUR HERSTELLUNG EINER BIOLOGISCHEN SUBSTANZ SOWIE DERARTIGE SUBSTANZ UND DEREN VERWENDUNG**
METHOD FOR PRODUCING A BIOLOGICAL SUBSTANCE, BIOLOGICAL SUBSTANCE AND THE USE THEREOF
PROCEDE DE PRODUCTION D'UNE SUBSTANCE BIOLOGIQUE, CETTE SUBSTANCE ET SON UTILISATION

(30) Priorität: 04.08.1998 DE 19834925
(43) Veröffentlichungstag der Anmeldung: 30.05.2001
(73) Patentinhaber: Gut Zum Leben Nahrungsmittel von Feld und Hof GmbH, 97828 Marktheidenfeld-Altfeld (DE)
(72) Erfinder: DELL'EVA, Marcello, D-97839 Esselbach (DE)
(74) Vertreter: Herden, Andreas F.
(86) Internationale Anmeldenummer: PCT/DE1999/002371
(87) Internationale Veröffentlichungsnummer: WO 2000/008121

(56) Entgegenhaltungen:
- EP-A- 0 475 510
- WO-A-92/20243
- WO-A-96/19121
- US-A- 3 969 514
- US-A- 4 515 818
- US-A- 4 639 374
- US-A- 5 667 838
- DATABASE WPI Section Ch, Week 199323 Derwent Publications Ltd., London, GB; Class D13, AN 1993-187491 XP002128686 & SU 1 741 726 A (PAVLOV V I), 23. Juni 1992 (1992-06-23)

## Beschreibung

Die Erfindung betrifft gemäß Anspruch 1 ein Verfahren zur Herstellung einer Substanz, die den vielfältigsten Zwecken dienen kann und insbesondere zur Weiterverarbeitung als Nahrungsmittel und zur Weiterverarbeitung als Körperpflege- oder Reinigungsmittel bestens geeignet ist. Ferner umfasst die Erfindung gemäß Anspruch 10 auch die durch das Verfahren erhaltliche Substanz sowie deren Verwendungen gemäß den Ansprüchen 17 und 18.

Insbesondere in der Nahrungsmittelherstellung werden heutzutage unterschiedliche biologische und chemische Stoffe eingesetzt. Bei vielen industriell verpackten Nahrungsmittelendprodukten besteht daher eine Bezeichnungspflicht für die Inhaltsstoffe, um den Verbraucher über deren Inhalt informiert zu halten, und insbesondere Allergie-gefährdeten Personen die Möglichkeit zur Meidung abträglicher Stoffe zu geben.

Bei anderen Nahrungsmittelendprodukten, insbesondere solchen, die nicht industriell verpackt sind, werden jedoch häufig die Inhaltsstoffe für den Verbraucher nicht vollständig erkenntlich.

Moderne Nahrungsmittelendprodukte enthalten eine Vielzahl von Grund- und Zusatzstoffen, die in den meisten Fällen zahlreiche Verarbeitungsschritte durchlaufen haben und den ursprünglichen natürlichen Nahrungsmitteln weit entrückt sind. Der Verbraucher kann sich trotz der Bezeichnungspflicht oftmals an den auf den Bezeichnungen aufgeführten Stoffen, die wiederum aus nicht bezeichneten zahlreichen Verarbeitungsschritten unterworfenen Stoffen aufgebaut sind, nicht orientieren. Dies gilt insbesondere auch für verwendete Konservierungsstoffe und genmanipulierte Stoffe, die eine immer weitere und unkontrollierte Verbreitung finden. In höchst nachteiliger Weise sind einige Nutzpflanzensorten, wie beispielsweise die Sojapflanze fast nur noch genmanipuliert zu erhalten.

Aus Database WPI Section Ch, Week 199323 Derwent Publications Ltd., London, GB; Class D13, AN 1993-187491, XP002128686 & SU 1 741 726 A, 23. Juni 1992 sind Süßwaren aus Karamel, Sonnenblumenmasse, Sonnenbluemnöl, Walnüssen und Zitronensaft bekannt, wobei das Vitamin C des Zitronensaftz lediglich als Antioxidant dient. Eine Mandelpaste zur Verwendung in Getränken oder Deserts ist in US-4,639,374 beschrieben. Harvey und Marylin Diamond beschreiben in einem Buch mit dem Titel: "Fit fürs Leben / Fit for Life 2", 3. Auflage 1992, Waldthausen Verlag, Ritterhude, ISBN 3-442-13621-0, eine sogenannte Almonaise, d.h. einen Mayonnaisenersatz mit Mandeln statt mit Eiern. Das Rezept der Almonaise enthält folgende Zutaten:

| | |
|---|---|
| Mandeln | 1/2 Tasse |
| Wasser | 1/2 Tasse |
| Knoblauch | kleine Scheibe |
| Meersalz | 1/2 Teelöffel (nach Wunsch) |
| salzfreies Gewürz | 1/2 Teelöffel |
| frischer Zitronensaft | 2 bis 3 Esslöffel |
| Cayennepfeffer | eine Prise |
| frisch gemahlener Pfeffer | nach Geschmack |
| Distelöl | 2 bis 3 Tassen |

Ferner werden historisch bedingt in unserem Zivilisationsraum zahlreiche Nahrungsmittel unter Verwendung von Kuhmilch, Kuhmilchprodukten und Hühnereiern hergestellt. Unter anderem ist in diesem Zusammenhang die Disposition zur allergischen Reaktion auf diese artfremden Eiweiße tierischer Herkunft bekannt. Kuhmilchprodukte, auch ökologisch hergestellte, und Hühnereier enthalten nicht unerhebliche Rückstände der den Tieren im industriellen Massenbetrieb verabreichten Medikamente und Hormone, die letzlieh dem Endverbraucher zugeführt werden. Weitere Allergene treten verursacht durch Tierfuttermittel auf. Außerdem weist Kuhmilch Aluminium und das selbst in geringen Dosen nicht unbedenkliche Blei auf.

Zur Bereitstellung von Nahrungs-, Pflege- und Reinigungsmitteln für Allergiker und Verbraucher, die medikamentfreie und hormonfreie Nahrungsmittel bevorzugen, sowie für Vegetarier ist es notwendig, die oben genannten Fremdeiweißprodukte durch eine möglichst natürliche, möglichst wenige industrielle Verarbeitungsschritte durchlaufende Substanz zu ersetzen.

Unter dem Gesichtspunkt der Transparenz der Inhaltsstoffe von Nahrungzmittelendprodukten für den Verbraucher sind Nahrungsmittel vorteilhaft, die aus einer kleinen Anzahl von ursprünglichen und natürlichen Zutaten mit möglichst wenigen industriellen Verarbeitungsschritten gefertigt werden.

Der Erfindung liegt folglich die Aufgabe zugrunde, eine Grundsubstanz für die Nahrungs-, Pflege- und Reinigungsmittelherstellung bereitzustellen, die aus ursprünglichen, natürlichen und nicht mit Genmanipulation in Kontakt gekommenen Stoffen besteht.

Diese Erfordernisse erfüllt die Erfindung in hohem Maße, wobei deren Substanzen darüber hinaus als vegane Produkte eingestuft werden können.

Die Erfindung erfüllt ferner die Aufgabe, ein wertigkeitsoptimiertes Nahrungsmittel mit sehr geringer Belastung des menschlichen Organismus bereitzustellen. Die patent gemäße Erfindung wird durch den Gegenstand der Ansprüche 1, 10, 17 und 18 definiert. Die patent gemäße Erfindung wird durch den Gegenstand der Ansprüche 1, 10, 17 und 18 definiert.

Erfindungsgemäß wird eine erste rührbare, insbesondere breiige Substanz aus der feinen Zerkleinerung oder Naßvermahlung von Kernen oder Nüssen unter Zugabe einer Flüssigkeit und insbesondere von Salz oder Zucker hergestellt. Diese erste Substanz wird unter Zugabe von Öl oder flüssig gemachtem Fett zu einer im wesentlichen flüssigen zweiten Substanz, wobei diese letztere durch Zugabe einer sauren Flüssigkeit eine festere Konsistenz erhält. Hierbei bestimmt die Menge der sauren Flüssigkeit die Konsistenz der letztlich hergestellten Substanz. Als Konsistenz soll hierbei entweder die Viskosität einer flüssigen bis breiigen Masse oder die Festigkeit einer zähen bis festen körperlichen Masse verstanden sein.

Die geringe Anzahl der Grund- und Inhaltsstoffe, sowie die einfachen mechanischen Verarbeitungsschritte lassen eine hohe Steuer- und Kontrollierbarkeit im Hinblick auf das Endprodukt zu. Rückstände von Medikamenten und Hormonen sowie genmanipulierten Zutaten können mit Sicherheit vermieden und die Zahl der potentiellen Allergene kann deutlich reduziert werden.

Die breiige erste Substanz besteht aus Kernen, speziell Sonnenblumenkernen, oder Nüssen, die fein zerkleinert werden, zu einer homogenen Masse oder naßgemahlen werden, bis im wesentlichen keine Körnchen mehr vorhanden sind.

Zu gleichen Teilen wird dann die breiige erste Substanz mit Öl oder flüssig gemachtem Fett verkuttert (vermengt), so daß eine zweite, in den meisten Fällen dickflüssige Substanz entsteht.

Das eingebrachte Öl ist vorzugsweise ein pflanzliches Öl und kann beispielsweise Sonnenblumen-, Oliven-, oder Distelöl sein. In weiterer alternativer Ausgestaltung kann jedoch auch ein beliebiges anderes verzehrbares Öl oder Fett, dessen geschmackliche oder Nahrungsbestandteile in der hergestellten erfindungsgemäßen Substanz erwünscht sind, verwendet werden.

Die Mengenverhältnisse sind in Bezug auf deren Gewichtsbestandteile bei der breiigen ersten Substanz folgendermaßen sein : Kerne (oder Nüsse) zu Flüssigkeit zu Salz bzw. Zucker etc. wie
100 : 50 bis 1000 : 0 bis 200

Soweit im nachfolgenden von speziellen Mengenbestandteilen ausgegangen wird, sind diese in Bezug auf deren Gewichtsanteile angegeben.

Es wurde festgestellt, daß je mehr von der ersten Flüssigkeit verwendet wurde, desto cremiger in der Regel auch die erste breiige Substanz wurde. Jedoch kann ein Punkt erreicht werden, bei welchem in Abhängigkeit von den Ausgangsstoffen und den jeweiligen Mengenverhältnissen keine Flüssigkeitsaufnahme mehr stattfindet und ein Ausflocken oder ein Verbleiben ungelöster Flüssigkeit auftreten kann.

Das Mengenverhältnis der breiigen ersten Substanz zu Öl oder flüssig gemachtem Fett kann in etwa 100 : 20 bis 300 betragen.

Die letztlich hergestellte Substanz, das heißt der Gegenstand der Erfindung, wird erzeugt durch Mischung der zweiten Substanz mit einer sauren Flüssigkeit. Als saure Flüssigkeit sind Säuerungsmittel, wie beispielsweise Säfte der Pflanzengattung Citrus, d.h. Zitronensaft oder Limonensaft oder ist die natürliche Säuerung durch Bakterien gut geeignet.

Verwendbar sind jedoch auch weitere, vornehmlich der Natur der Erfindung entsprechende, natürliche oder naturnahe saure Flüssigkeiten oder Säuerungsmittel. Das Mengenverhältnis kann bei der Mischung der sauren zweiten Flüssigkeit zur flüssigen zweiten Substanz in etwa 2 bis 20 : 100 betragen. Die letztlich hergestellte Substanz hat einen pH-Wert im leicht sauren Milieu.

Bei der Einmischung der sauren zweiten Flüssigkeit oder Säuerungsmittel findet eine Reaktion statt, die zu einer geänderten Viskosität, d.h. einer festeren Substanz führt. Diese Reaktion tritt erstaunlicherweise schon bei tropfenweiser Zugabe saurer Flüssigkeit ein. Diese festere Substanz kann mit Luft oder Stickstoff im begrenzten Masse aufgeschlagen werden.

Aus dem Stadium der noch gießbaren zweiten Substanz erfolgt mit zunehmender Zugabe der Übergang zum pasteusen, noch nicht festem Zustand mit einer Konsistenz ähnlich der von Quark oder Frischkäse und einer helleren Farbe und sämiger Konsistenz.

Eine spezielle und bevorzugte Ausführungsform wird nachfolgend erläutert.

100 Gramm Sonnenblumenkerne werden durch Verkuttern fein zerkleinert oder naß gemahlen, bis keine Körnchen mehr vorhanden sind. Die Temperatur bei diesem Zerkleinerungsprozeß ist bevorzugt etwa 20 Grad Celsius. Es entsteht dabei eine homogene Masse, in die 165 Gramm Wasser und 8 Gramm Salz hineingemischt werden, so daß eine rührbare, insbesondere breiige, Substanz entsteht. In diese breiige Substanz wird 200 Gramm Sonnenblumenöl eingekuttert. Es entsteht eine helle graue dickflüssige Masse.

Der nächste Arbeitsschritt sieht die Einmengung von 40 Gramm frischem Zitronensaft vor. Die dickflüssige Masse reagiert dabei zu einer hellen pasteusen Substanz ähnlicher Konsistenz wie Quark oder Frischkäse, leicht gelockert durch beim Mischvorgang eingeschlagene Luft.

Diese Substanz erwies sich als hervorragend geeignet als Ersatz- oder Grundstoff für im wesentlichen alle festen und flüssigen Nahrungsmittel, bei welchen bisher Milch- und Milcheiweißprodukte, wie beispielsweise Sahne, Quark, Käse oder dergleichen Verwendung fand.

Bereits diese Substanz stellt einen frei handelbaren eigenständigen Grundstoff dar, welcher vielfältigen Verwendungszwecken zugeführt werden kann.

Soll diese Substanz längerfristig haltbar sein, kann deren pH-Wert auf vorzugsweise etwa 4,5 eingestellt und/oder eine Pasteurisierung vorgenommen werden.

Wird die erfindungsgemäße Substanz beispielsweise als Getränk weiterverarbeitet, so ist ein Anteil einer Aroma-gebenden Substanz, wie etwa in Form von Fruchtsaftbestandteilen, geeignet, um Ersatz für die herkömmlichen Milchmischgetränke bereitzustellen.

Je nach Würzung der hergestellten Substanz kann diese auch bei entsprechend eingestellter Festigkeit als Käse-, Wurst- oder Fleischersatz- oder -zugabestoff verwendet werden.

Werden süße Aromastoffe beigegeben können Konditoreiwaren, wie beispielsweise Käse- oder Sahnetorten bzw. Törtchen und dergleichen hergestellt werden. Auch Joghurt-, Quark- und Frischkäseprodukte können bei geeigneter Einstellung der Viskosität durch Verwendung der erfindungsgemäßen Substanz hergestellt werden.
In überraschender Weise stellte sich heraus, daß auch Körperpflegemittel mit sehr geringem Anteil von Allergenen auf Basis der hergestellten Substanz angefertigt werden konnten. So können Cremes, Sonnen-Milch und dergleichen durch Beigabe entsprechender Duft-gebender Stoffe, bevorzugterweise in Form von Kräuter- oder Pflanzenbestandteilen, bereitgestellt werden.

Auch Reinigungsmittel konnten mit der erfindungsgemäßen Substanz hergestellt werden, wobei diese in bevorzugter Weise dann auch einen körnigen Bestandteil aufwiesen.

Der körnige Bestandteil kann grobvermahlene Sonnenblumenkerne oder -Schrot, Holz-, Kokosspäne, Sand oder Kalkbestandteile umfassen und dient im wesentlichen einem zusätzlichen mechanischen Reinigungsvorgang.

Obwohl die Erfindung vorstehend anhand bevorzugter Ausführungsformen beschrieben wurde, ist diese nicht darauf beschränkt.

Es kann beispielsweise die Reihenfolge der Verfahrensschritte abgeändert werden oder können jeweils an Stelle von Produkten einer Sorte, wie beispielsweise Kernen oder Säften nur einer Pflanzen- oder Fruchtsorte Mischungen von Kernen und Säften mehrerer Pflanzen- oder Fruchtsorten eingesetzt werden. Ferner ist nicht nur der reine Ersatz von Milcheiweißprodukten, sondern auch eine Zugabe zu diesen oder ein Teilersatz möglich.

Neben Milcheiweißprodukten können aber auch beispielsweise Tofu-enthaltende Produkte, sowie Tofu selbst ersetzt werden.

Die Fettmenge und Fettbeschaffenheit (Festigkeit des Fettes) bestimmen generell die Konsistenz der Substanz soweit, daß sich Konsistenz von schneidfester Masse so wie beispielsweise der griechische Feta-Käse bis zu einer Flüssigkeit einstellen oder variieren läßt.

Die Konsistenz, d.h. die Festigkeit des erfindungsgemäßen Stoffs lässt sich jedoch zusätzlich zur Säure auch eben über den Gehalt an Fett oder die Wahl des Fettes bestimmen; die Menge und Beschaffenheit des Fettes dient dann auch zur Festigkeits- oder Konsistenzeinstellung.

Die Säure bzw. das hinzugegebene Säuerungsmittel koaguliert Eiweiß durch eine pH-Wert-Absenkung. Dies bedeutet, daß maximale Festigkeit auch durch eine bestimmte Anzahl von H+-Ionen bereitgestellt werden kann. Die maximale Festigkeit liegt ungefähr bei einem pH-Wert = 4,0 denn es tritt in der Regel keine weitere Verfestigung bei weiterer Säurezugabe oberhalb von pH = 4,0 auf. Generell gilt jedoch unterhalb dieses Wertes, daß je mehr Säure hinzugegeben wird, desto höhere Festigkeit erreicht wird. Der pH-Wert sollte aber maximal gleich 5 sein, da sonst kein weiteres Binden erreicht werden kann..

Zur einfacheren Konsistenzeinstellung lässt sich folgende Tabelle aufstellen:

| | |
|---|---|
| höhere Schneidfestigkeit | flüssigere Konsistenz |
| mehr Säure | weniger Säure |
| mehr Fett | weniger Fett |
| wenig Wasser | mehr Wasser |

Eine Emulsion entsteht jedoch nur, in einem pH-Bereich < 5, d.h. es ist gegebenenfalls eine Mindestzugabe an Säure notwendig, um diesen Bereich sicherzustellen.

Nachfolgend werden weitere bevorzugte Ausführungsbeispiele angegeben.

Hierbei soll der Wasseranteil im Bereich von 45 % bis 25 % liegen, da sich andernfalls eventuell keine Emulsion einstellt.

Der Anteil an zugesetztem Fett soll zwischen etwa 30 % bis 50 % liegen.

Eine besonders bevorzugte Ausführungsform umfasst an:

| | |
|---|---|
| Sonnenblumenanteil etwa | 20 % Gewichtsprozent |
| an Flüssigkeit | 54 % Gewichtsprozent |
| vorzugsweise bestehend aus | |
| zugesetztem Wasser | 47 % Gewichtsprozent und |
| Zitronensaft mit etwa | 7 % Gewichtsprozent |

| | |
|---|---|
| zugesetztem Fett | 26 % |

Die flüssige Variante kann beispielhaft auch durch die bevorzugten Verhältnisse formuliert werden.
Sonnenblumen : zugegebene wässrige Flüssigkeit: zugesetzte Fette oder Öle im Verältnis 3 : 8 : 4 bei einem pH = 4,5.

Die festeste Variante kann beispielhaft auch durch die bevorzugten Verhältnisse wie folgt angegeben werden. Sonnenblumen : zugegebene wässrige Flüssigkeit: zugesetzte Fette oder Öle im Verhältnis 3: 4 : 8 und pH = 4.

Die Erfahrung mit erfindungsgemäßem Milchersatz lehrt, daß die Emulsion unter den oben genannten Grenzen fester oder flüssiger angesetzt werden kann. Der Emulgieprozeß muß vollendet sein, dann kann dieses Produkt mit Wasser oder wässriger Flüssigket zu Milch gestreckt werden.

Als Kerne dienen bevorzugt, jedoch ohne Beschränkung der Allgemeinheit, Sonnenblumenkerne, Mandeln, Haselnüsse, Walnüsse sortenrein sowie beliebige Mischungen von diesen.

Als Öle und Fette dienen bevorzugt, jedoch ohne Beschränkung der Allgemeinheit, Avokadoöl, Distelöl, Rapsöl, Sonnenblumenöl, Mandelöl, Sojaöl, Kürbiskernöl, jeweils sortenrein oder in beliebigen Mischungsverhältnissen.

Soweit nachfolgend Prozentangaben gemacht werden, sind diese jeweils als Gewichtsprozent zu verstehen.

Ein erfindungsgemäßes Basisprodukt, welches vom Anmelder den Markennamen Ibi verliehen bekam, erhält man auch mit Sonnenblumenkernen fein gemahlen (20 %), Wasser (33,3 %), Salz (0,7 %), Sonnenblumenöl (40 %), Zitronensaft (6 %). Wasser, Sonnenblumenöl und Salz können variiert werden, je nach gewünschter Konsistenz. Alle Zutaten werden der Reihe nach zugefügt und miteinander vermischt oder geschlagen. Danach pasteurisieren.

Das erfindungsgemäße Nahrungsmittel kann mit allen Süßstoffen gesüßt werden: raffiniertem Zucker, Agavendicksaft, Apfeldicksaft, Honig.
Bevorzugte Anteile: erfindungsgemäßer Grundstoff Ibi, wie vorstehend beschrieben, (85 %), Süßstoff (15 %).

### Seife, Shampoo, Zahnpasta erhält man aus Ibi wie folgt:

Ibi Basis- oder Grundstoff wie vorstehend beschrieben, Teebaumöl (1 Tropfen pro 10g Ibi Basis- oder Grundstoff) zusammen mischen, danach pasteurisieren. Als Reibemittel zur mechanischen Reinigung eignen sich neben anderen folgende Stoffe: Salz, Getreidekleie. Diese Stoffe werden jeweils nach Wunsch zugefügt. Teebaumöl ist ein natürliches Haltbarkeitsmittel. Die Haltbarkeit kann weiter verbessert werden durch zugelassene und allgemein bekannte Konservierungsstoffe.

Anstelle von Sonnenblumenöl für die Ibi-Basis, d.h. den Ibi-Grundstoff eignen sich auch folgende Öle: Avocadoöl, Mandelöl, Jojobaöl.

### IBi Milch

Ibi Basis- oder Grundstoff wie vorstehend beschrieben mit Wasser im Verhältnis 2 : 7 mixen. Eine besonders bevorzugte Verfeinerungsmöglichkeit verwendet Vanille pur, Cacao, Mandeln fein gemahlen (bis max. 2 %).

Diese Ausführungsbeispiele zeigen dem Fachmann eindrücklich die breite und vielfältige Einsetzbarkeit der erfindungsgemäßen Substanz, sind jedoch nicht geeignet die Allgemeinheit der Erfindung zu beschränken.

Die Erfindung leistet somit einen wesentlichen, wichtigen und grundlegenden Beitrag für ein weniger mit chemischen Stoffen oder genveränderten Produkten belastetes Leben. Darüber hinaus können auch allergisch belastete Personen wieder an vielen Freuden eines normalen Lebens teilhaben, die Ihnen bislang durch Ihre Krankheit verwehrt waren, denn die jeweils sortenreine Herstellung der erfindungsgemäßen Substanzen gestatten den Ausschluß ungewollter Allergene mit der nötigen hohen Sicherheit.

## Patentansprüche

1. Verfahren zur Herstellung einer Substanz, wobei
- eine rührbare, insbesondere breiige, erste Substanz aus der Zerkleinerung von Kernen oder Nüssen
- unter Zugabe einer ersten Flüssigkeit, und vorzugsweise unter Zugabe von Salz, oder Zucker hergestellt wird,
- dieser rührbaren, insbesondere breiigen, ersten Substanz Öl oder flüssiggemachtes Fett hinzugegeben wird und somit eine zweite Substanz gewonnen wird, **dadurch gekennzeichnet, dass**
- dieser zweiten Substanz eine saure zweite Flüssigkeit oder ein Säuerungsmittel zugegeben wird, wodurch eine festere Konsistenz erhalten wird, wobei durch die Menge der zugegebenen sauren zweiten Flüssigkeit oder des Säuerungsmittels die Konsistenz der hergestellten Substanz bestimmbar ist,
wobei die Mengenverhältnisse der Kerne oder Nüße zu der ersten Flüssigkeit zu Salz bzw. Zucker in Bezug auf deren Gewichtsbestandteile 100:50 bis 1000:0 bis 200 betragen und die letztlich hergestellete Substanz einen pH-Wert kleiner oder gleich 5 aufweist und wobei der Anteil an zugesetzten Öl oder Fett zwischen 13,9 und 50 Gewichtsprozent beträgt.

2. Verfahren nach Anspruch 1,
**dadurch gekennzeichnet,**
**dass** die zweite Substanz durch Bakterien gesäuert wird.

3. Verfahren nach Anspruch 1 oder 2,
**dadurch gekennzeichnet,**
**daß** das zugegebene Öl oder Fett aus Kernen oder Nüssen gewonnen ist.

4. Verfahren nach einem der Ansprüche 1 bis 3,
**dadurch gekennzeichnet,**
**daß** die Kerne oder Nüsse von einer einzigen Pflanzenart stammen.

5. Verfahren nach Anspruch 4,
**dadurch gekennzeichnet,**
**daß** die Kerne Sonnenblumenkerne sind.

6. Verfahren nach einem der Ansprüche 1 bis 5,
**dadurch gekennzeichnet,**
**daß** die erste Flüssigkeit Wasser, Obst- oder Gemüsesaft umfasst oder aus Pflanzen oder Pilzen, gewonnen ist.

7. Verfahren nach einem der Ansprüche 1 bis 6,
**dadurch gekennzeichnet,**
**daß** die saure zweite Flüssigkeit Zitronensaft ist.

8. Verfahren nach einem der Ansprüche 1 bis 7,
**dadurch gekennzeichnet,**
**daß** das Mengenverhältnis der sauren zweiten Flüssigkeit zur flüssigen zweiten Substanz in etwa 2 bis 20 : 100 in Bezug auf deren Gewichtsanteile beträgt.

9. Verfahren nach einem der Ansprüche 1 bis 8,
**dadurch gekennzeichnet,**
**daß** die hergestellte Substanz mit Luft oder Stickstoff etwas aufgeschlagen wird.

10. Substanz, **erhältlich nach dem Verfahren nach einem der vorstehenden Ansprüche,** hergestellt aus
- einer rührbaren ersten Substanz aus zerkleinerten Kernen oder Nüssen
- einer ersten Flüssigkeit und vorzugs weise Salz oder Zucker,
- Öl oder flüssiggemachtem Fett und
- einer sauren zweiten Flüssigkeit oder einem Säuerungsmittel, durch deren bzw. dessen zugegebener Menge die Konsistenz der hergestellten Substanz bestimmbar ist,
wobei die Mengenverhältnisse der Kerne oder Nüsse zu der ersten Flüssigkeit zu Salz bzw. Zucker in Bezug auf deren Gewichtsbestandteile 100 : 50 bis 1000 : 0 bis 200 betragen, und die Substanz einen pH-Wert kleiner oder gleich 5 aufweist und aufgrund der zugegebenen sauren zweiten Flüssigkeit bzw. des Säuerungsmittels eine geänderte Konsistenz besitzt, derart dass die Substanz verfestigt ist **und wobei der Anteil an zugesetztem Öl oder Fett zwischen 13,9 und 50 Gewichtsprozent beträgt.**

11. Substanz nach Anspruch 10,
**dadurch gekennzeichnet,**
**daß** Eiweiß aufgrund der pH-Wert-Absenkung durch das hinzugegebene Säuerungsmittel koaguliert ist.

12. Substanz nach Anspruch 10 oder 11,
**dadurch gekennzeichnet,**
**daß** das zugegebene Öl Avokadoöl, Distelöl, Rapsöl, Sonnenblumenöl, Mandelöl, Sojaöl, Kürbiskernöl oder Jojobaöl ist.

13. Substanz nach einem der Ansprüche 10 bis 12,
**dadurch gekennzeichnet,**
**daß** die Kerne Sonnenblumenkerne sind.

14. Substanz nach einem der Ansprüche 10 bis 13,
**dadurch gekennzeichnet,**
**daß** die erste Flüssigkeit Wasser, Obst- oder Gemüsesaft umfasst oder aus Pflanzen oder Pilzen, gewonnen ist.

15. Substanz nach Anspruch 14,
**dadurch gekennzeichnet,**
**daß** der Wasseranteil im Bereich von 45 % bis 25 % liegt.

16. Substanz nach einem der Ansprüche 10 bis 15,
**dadurch gekennzeichnet,**
**daß** die saure zweite Flüssigkeit Zitronensaft ist.

17. Verwendung der Substanz nach einem der Ansprüche 10 bis 16 als Bestandteil eines Nahrungsmittels.

18. Verwendung der Substanz nach einem der Ansprüche 10 bis 16 als Tofu- oder Milcheiweißersatzprodukt.

## Claims

1. A method for producing a substance, wherein:
- a stirrable, in particular pasty first substance is produced by comminuting seeds or nuts
- while adding a first liquid, and preferably by adding salt or sugar;
- oil or liquefied fat is added to this stirrable, in particular pasty first substance, thus producing a second substance;
**characterised in that**
- an acidic second liquid or an acidification agent is added to said second substance so that a more solid consistency is obtained, the consistency of the produced substance being determinable by the quantity of added acidic second liquid or acidification agent, wherein the relative proportions of seeds or nuts to the first liquid with respect to the parts by weight thereof to salt or sugar are 100 : 50-1000 : 0-200, and wherein the finally produced substance has a pH value of less than or equal to 5, and wherein the proportion of added oil or fat is from 13.9 to 50 percent by weight.

2. The method according to claim 1,
**characterised in that**
the second substance is acidified by bacteria.

3. The method according to claim 1 or 2,
**characterised in that**
the added oil or fat is obtained from seeds or nuts.

4. The method according to any one of claims 1 to 3, **characterised in that**
the seeds or nuts originate from a single type of plant.

5. The method according to claim 4,
**characterised in that**
the seeds are sunflower seeds.

6. The method according to any one of claims 1 to 5, **characterised in that**
the first liquid comprises water, fruit juice or vegetable juice, or is obtained from plants or fungi.

7. The method according to any one of claims 1 to 6, **characterised in that**
the acidic second liquid is lemon juice.

8. The method according to any one of claims 1 to 7, **characterised in that**
the relative proportions of the acidic second liquid to the liquid second substance are about 2-20 : 100 in percent by weight thereof.

9. The method according to any one of claims 1 to 8, **characterised in that**
the produced substance is beaten up to some extent with air or nitrogen.

10. A substance, obtainable by a method according to any one of the preceding claims, produced from
- a stirrable first substance from comminuted seeds or nuts;
- a first liquid and preferably salt or sugar;
- oil or liquefied fat; and
- an acidic second liquid or an acidification agent, wherein the consistency of the produced substance can be determined through the added quantity thereof;
wherein the relative proportions of the seeds or nuts to the first liquid to salt or sugar with respect to the parts by weight thereof are 100 : 50-1000 : 0-200, and wherein the substance has a pH value of less than or equal to 5 and as a result of the added acidic second liquid or the acidification agent has a changed consistency such that the substance is solidified, and wherein the proportion of added oil or fat is from 13.9 to 50 percent by weight.

11. The substance according to claim 10,
**characterised in that**
protein is coagulated by the added acidification agent as a result of the reduction of the pH value.

12. The substance according to claim 10 or 11,
**characterised in that**
the added oil is avocado oil, thistle oil, rapeseed oil, sunflower oil, almond oil, soya oil, marrow oil or jojoba oil.

13. The substance according to any one of claims 10 to 12, **characterised in that**
the seeds are sunflower seeds.

14. The substance according to any one of claims 10 to 13, **characterised in that**
the first liquid comprises water, fruit juice or vegetable juice, or is obtained from plants or fungi.

15. The substance according to claim 14,
**characterised in that**
the water content is in a range from 45 to 25 %.

16. The substance according to any one of claims 10 to 15, **characterised in that**
the acidic second liquid is lemon juice.

17. Use of the substance according to any one of claims 10 to 16 as an ingredient of a food.

18. Use of the substance according to any one of claims 10 to 16 as a tofu or milk protein substitute product.

## Revendications

1. Procédé de production d'une substance, dans lequel :
- une première substance pouvant être agitée, en particulier à consistance pâteuse, à partir du broyage de graines ou de noix
- avec addition d'un premier liquide et, avantageusement, avec addition de sel ou de sucre, est préparée,
- à cette première substance en particulier à consistance pâteuse, pouvant être agitée, est ajoutée une huile ou une graisse fluidifiée, et ainsi une seconde substance est obtenue,
**caractérisé en ce que**
- à cette seconde substance un second liquide acide ou un agent acidifiant est ajouté, ce qui permet d'obtenir une consistance plus ferme, la consistance de la substance produite pouvant être réglée par la quantité ajoutée de second liquide acide ou d'agent acidifiant,
dans lequel les rapports quantitatifs des graines ou noix relativement au premier liquide et au sel et/ou au sucre ont des valeurs sur base pondérale d'environ 100:50 à 1000:0 à 200 et la substance finalement produite a une valeur de pH inférieure ou égale à 5 et dans lequel la proportion d'huile ou de graisse ajoutée est comprise entre 13,9 et 50 % en poids.

2. Procédé suivant la revendication 1, **caractérisé en ce que** la seconde substance est acidifiée par des bactéries.

3. Procédé suivant la revendication 1 ou 2, **caractérisé en ce que** l'huile ou la graisse ajoutée est extraite de graines ou de noix.

4. Procédé suivant l'une des revendications 1 à 3, **caractérisé en ce que** les graines ou les noix proviennent d'une seule espèce végétale.

5. Procédé suivant la revendication 4, **caractérisé en ce que** les graines sont des graines de tournesol.

6. Procédé suivant l'une des revendications 1 à 5, **caractérisé en ce que** le premier liquide comprend de l'eau, un jus de fruit ou de légume ou est extrait de plantes ou de champignons.

7. Procédé suivant l'une des revendications 1 à 6, **caractérisé en ce que** le second liquide acide est du jus de citron.

8. Procédé suivant l'une des revendications 1 à 7, **caractérisé en ce que** le rapport quantitatif sur base pondérale du second liquide acide à la seconde substance liquide est d'environ 2 à 20:100.

9. Procédé suivant l'une des revendications 1 à 8, **caractérisé en ce que** la substance produite est légèrement battue avec de l'air ou de l'azote.

10. Substance, pouvant être obtenue selon le procédé suivant l'une des revendications précédentes, produite à partir
- d'une première substance pouvant être agitée, obtenue à partir de graines ou de noix broyées,
- d'un premier liquide et de préférence de sel ou de sucre,
- d'une huile ou d'une graisse fluidifiée, et
- d'un second liquide acide ou d'un agent acidifiant, dont la quantité ajoutée permet de régler la consistance de la substance produite,
dans laquelle les rapports quantitatifs des graines ou noix relativement au premier liquide et au sel et/ou au sucre s'élèvent à 100:50 à 1000:0 à 200 sur base pondérale, et la substance a une valeur de pH inférieure ou égale à 5 et possède une consistance modifiée par l'addition du second liquide acide ou de l'agent acidifiant, de façon telle que la substance est rendue plus ferme, et dans laquelle la proportion d'huile ou de graisse ajoutée est comprise entre 13,9 et 50 % en poids.

11. Substance suivant la revendication 10, **caractérisée en ce que** l'albumine est coagulée par l'agent acidifiant ajouté en raison de l'abaissement du pH.

12. Substance suivant la revendication 10 ou 11, **caractérisée en ce que** l'huile ajoutée est de l'huile d'avocat, de carthame, de colza, de tournesol, d'amande, de soja, d'une cucurbitacée ou de jojoba.

13. Substance suivant l'une des revendications 10 à 12, **caractérisée en ce que** les graines sont des graines de tournesol.

14. Substance suivant l'une des revendications 10 à 13, **caractérisée en ce que** le premier liquide comprend de l'eau, du jus de fruit ou de légume ou est extrait de plantes ou de champignons.

15. Substance suivant la revendication 14, **caractérisée en ce que** la proportion d'eau est dans la plage de 45 % à 25 %.

16. Substance suivant l'une des revendications 10 à 15, **caractérisée en ce que** le second liquide acide est du jus de citron.

17. Utilisation de la substance suivant l'une des revendications 10 à 16 comme constituant d'un produit alimentaire.

18. Utilisation de la substance suivant l'une des revendications 10 à 16 comme produit de substitution du tofu ou des protéines du lait.
